# EUROPEAN PATENT APPLICATION

(11) **EP 4 148 629 A1**
(43) Date of publication of application: **15.03.2023**
(21) Application number: 21818809.2
(22) Date of filing: 03.06.2021
(51) Int. Cl.: G06N 3/08, G06N 3/04

(54) **NEURAL NETWORK TRAINING METHOD VIA AUTO-ENCODER AND MULTIPLE INSTANCE LEARNING, AND COMPUTING SYSTEM FOR PERFORMING SAME**

(30) Priority: 05.06.2020 KR 20200068640
(71) Applicant: Deep Bio Inc., Seoul 08380 (KR)
(72) Inventor: KIM, Sun Woo, Seongnam-si, Gyeonggi-do 13599 (KR); KWAK, Tae Yeong, Seoul 05119 (KR); MUN, Ye Chan, Seoul 08393 (KR)
(74) Representative: Flügel Preissner Schober Seidel
(86) International application number: PCT/KR2021/006966
(87) International publication number: WO 2021/246810

(57) **Abstract**

The present disclosure relates to a neural network training method and a computing system for performing the same, and more specifically, the neural network training method, which may enhance performance of a neural network even with little training data and the computing system for performing the same by using an auto-encoder and a multiple instance training method and extracting many data instances from one data bag as instances for training.

## Description

### TECHNICAL FIELD

The present disclosure relates to a neural network training method and a computing system for performing the same, and more specifically, the neural network training method, which may enhance performance of a neural network even with little training data using an auto-encoder and a multiple instance training method, and the computing system for performing the same.

### BACKGROUND ART

Recently, a try to automate work such as recognition or classification of images by a computer system due to developments of mechanical training is actively made. In particular, a try to automate various classification or determination which is performed by a human using the neural network(e.g., a deep training method which uses Convolution Neural Network(CNN)) which is a type of mechanical training is made. To perform diagnosis which reads a biometric image(e.g., a biological tissue slide of a patient) using deep training which uses the neural network(e.g., CNN)and determines a status or a symptom about a specific disease may be a representative example.

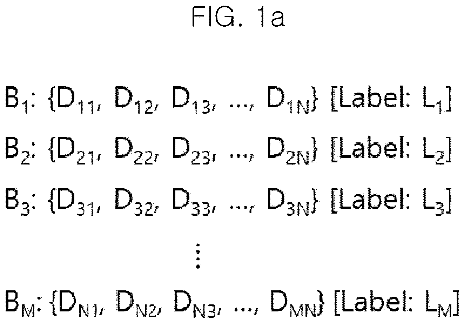

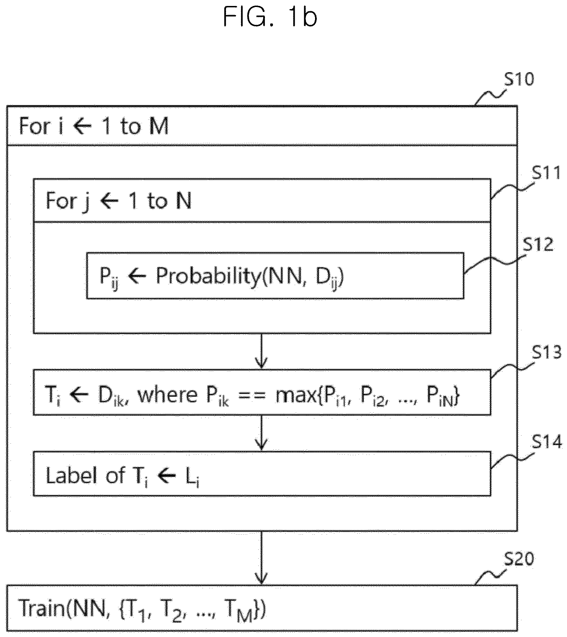

Meanwhile, referring to FIGS. 1A and 1B, multiple instance training which is one of background arts of the present disclosure is described.

Differently from individual instance training which uses one instance as a training unit, multiple instance training considers a bag which is a set of instances as a training unit. Accordingly, the individual instance training labels an instance, otherwise the multiple instance training label a bag other than the instance. The multiple instance training is similar with the individual instance training except an aspect of the training unit but additionally has the following limitations. When performing binary classification, it is assumed that if a bag is positive, at least one or more of instances which are present in the bag is positive, and if the bag is negative, all instances in the bag are negative.

Due to these features, the multiple instance training may apply to a field which diagnoses a lesion from a pathological whole slide image using, for example, a neural network. This is because in order to learn the neural network for diagnosing the lesion, image patches into which the image is segmented in a certain size other than the whole slide image are used as the training data but information(i.e., a label) about whether there is a lesion is given to the whole slide

image other than a patch unit. FIG. 1A is a view illustrating an example of training data used in the multiple instance training. FIG. 1 illustrates M bags B₁ to B_{M} including N data instances, respectively. In FIG. 1, the bag Bᵢ is labeled with Lᵢ(here, i is any integer where 1<=i<=M), a data instance Dᵢⱼ presents a j^{th} instance included in the bag Bᵢ(here, j is any integer where 1<=j<=N).

FIG. 1B is a view illustrating pseudo code which presents an example of a process of training a neural network(NN) via a multiple instance training technique. FIG. 1B presents a process of training 1 epoch of training data, wherein many epochs of training may be proceeded in a real training process. FIG. 1B assumes that training is proceeded with training data shown in FIG. 1A.

Referring to FIG. 1B, in the multiple instance training, a process of extracting training data instances T₁ to T_{M} from each bag B₁ to B_{M} is performed at first(S10), and then a process of training the neural network(NN) with the extracted training data instances is performed(S20).

When more specifically explaining the step(S10), the following process is performed for the bag Bᵢ(here, i is any integer where 1<=i<=M).

Each data instance within the bag Bᵢ of the neural network(NN) where training is proceeded at present is inputted, and probabilities where the relevant instance is positive(for example, the lesion is present) are calculated(S11, S12).

A data instance (Dᵢₖ) where positive probabilities of the relevant data instance of all data instances within the bag (Bᵢ) are the biggest is determined as the training data instance(Tᵢ)(S13), and a label of the training data instance (Tᵢ) becomes the label given to the bag (Bᵢ)(S14).

Since the conventional multiple instance training described above extracts one instance for one bag and uses it for training, there is a problem that many bags are needed in order to enhance performance of the neural network. For example, in order to use the existing multiple instance training in the neural network for detecting the lesion, many whole slide images to which indications about whether there is a lesion are given are needed.

Also, as previously described, since in the multiple instance training, the neural network before completing training is used in a process of extracting a training data instance, if many data instances are extracted from one bag, there is a problem that probabilities where a wrong instance is extracted become high.

### DISCLOSURE OF INVENTION

### TECHNICAL PROBLEM

A technical problem to be resolved by the present disclosure is to extract many data instances, instances for training, from one data bag, thereby providing a method and a system for properly training the neural network even with relatively little data.

### TECHNICAL SOLUTION

According to one aspect of the present disclosure, provided is a neural network training method performed in a computing system which includes an auto-encoder configured to determine whether an inputted data instance is in a first state or a second state and a neural network which may output probabilities where the inputted data instance is in the first state or the second state, wherein the neural network training method includes an extracting step of extracting an instance for training which is part of data instances included in a data bag, for each of a plurality of data bags labeled with any one of the first state or the second state; and a training step of training the neural network based on an instance for training corresponding to each of the plurality of data bags, wherein the extracting step includes a step of inputting each data instance included in the data bag to the neural network in training and calculating probabilities for each data instance included in the data bag; and a step of determining part of each data instance included in the data bag as an instance for training based on probabilities for each data instance included in the data bag and a determination result of the auto-encoder with respect to at least part of each data instance included in the data bag.

In an example, the neural network training system may determine whether the data instance inputted to the auto-encoder is in the first state or the second state, based on a difference between a data instance inputted to the auto-encoder and output data outputted from the auto-encoder.

In an example, the auto-encoder is pre-trained only with a data instance which is in the first state, wherein the step of determining part of each data instance included in the data bag as an instance for training based on probabilities for each data instance included in the data bag and the determination result of the auto-encoder with respect to at least part of each data instance included in the data bag may include a step of if the data bag is labeled with the first state, inputting data instances to the auto-encoder in the order from a data instance which has the highest probabilities where it is in the second state to a data instance which has the lowest probabilities where it is in the second state and determining top part of data instances determined by the auto-encoder as being in the first state, as an instance for training corresponding to the data bag; and a step of if the data bag is labeled with the second state, inputting data instances to the auto-encoder in the order from a data instance which has the highest probabilities where it is in the second state to a data instance which has the lowest probabilities where it is in the second state and determining top part of data instances determined by the auto-encoder as being in the second state, as an instance for training corresponding to the data bag.

In an example, the neural network training method may further include a step of repetitively performing 1 epoch or more of the extracting step and the training step.

In an example, each of the plurality of data bags is a whole image and a data instance included in each of the plurality of data bags is each image patch into which the whole image corresponding to the data bag is segmented in a certain size.

According to one of the other aspects of the present disclosure, provided is a neural network training method performed in a computing system which includes an auto-encoder configured to determine whether an inputted patch is in a first state or a second state-here, the patch is one of those into which an image is segmented in a certain size-; and a neural network which may output probabilities where the inputted patch is in the first state or the second state, wherein the neural network training method includes an extracting step of extracting a patch for training which is part of patches forming an image for training, for each of a plurality of images for training labeled with any one of the first state or the second state; and a training step of training the neural network based on the patch for training corresponding to each of the plurality of images for training, wherein the extracting step includes a step of inputting each patch forming the image for training to the neural network in training, and calculating probabilities for each patch forming the image for training; and a step of determining part of each patch forming the image for training as a patch for training based on probabilities for each patch forming the image for training and a determination result of the auto-encoder with respect to at least part of each patch forming the image for training.

In an example, the auto-encoder is pre-trained only with a patch which is in the first state, wherein the step of determining part of each patch forming the image for training as a patch for training based on probabilities for each patch forming the image for training and the determination result of the auto-encoder with respect to at least part of each patch forming the image for training may include a step of if the image for training is labeled with the first state, inputting patches to the auto-encoder in the order from a patch which has the highest probabilities where it is in the second state to a patch which has the lowest probabilities where it is in the second state and determining top part of patches determined by the auto-encoder as being in the first state, as a patch for training corresponding to the image for training; and a step of if the image for training is labeled with the second state, inputting patches to the auto-encoder in the order from a patch which has the highest probabilities where it is in the second state to a patch which has the lowest probabilities where it is in the second state and determining top part of patches determined by the auto-encoder as being in the second state, as a patch for training corresponding to the image for training.

In an example, each of images for training may be any one of an image including a lesion due to a certain disease or an image not including the lesion, the first state may be a normal state where the lesion is not present, and the second state may be an abnormal state where the lesion is present.

According to one of the other aspects of the present disclosure, provided is a computer program recorded on a medium installed in a data processing device and for performing the above method.

According to one of the other aspects of the present disclosure, provided is a computer readable recording medium on which a computer program for performing the above method is recorded.

According to one of the other aspects of the present disclosure, the computing system includes a processor and a memory, wherein provided is the computing system configured such that if the memory is performed by the processor, the computing system performs the above method.

According to one of the other aspects of the present disclosure, provided is a neural network training system includes a storage module which stores an auto-encoder configured to determine whether an inputted data instance is in a first state or a second state and a neural network which may output probabilities where the inputted data instance is in the first state or the second state; an extraction module of extracting an instance for training which is part of data instances included in a data bag, for each of a plurality of data bags labeled with any one of the first state or the second state; and a training module of training the neural network based on the instance for training corresponding to each of the plurality of data bags, wherein the extraction module inputs each data instance included in the data bag to the neural network in training, calculates probabilities for each data instance included in the data bag, and determines part of each data instance included in the data bag as an instance for training based on probabilities for each data instance included in the data bag and a determination result of the auto-encoder with respect to at least part of each data instance included in the data bag.

In an example, the extraction module may determine whether the data instance inputted to the auto-encoder is in the first state or the second state, based on a difference between a data instance inputted to the auto-encoder and output data outputted from the auto-encoder.

In an example, the auto-encoder is pre-trained only with a data instance which is in the first state, wherein in order to determine part of each data instance included in the data bag as an instance for training based on probabilities for each data instance included in the data bag and the determination result of the auto-encoder with respect to at least part of each data instance included in the data bag, if the data bag is labeled with the first state, the extraction module may input data instances to the auto-encoder in the order from a data instance which has the highest probabilities where it is in the second state to a data instance which has the lowest probabilities where it is in the second state and determine top part of data instances determined by the auto-encoder as being in the first state, as an instance for training corresponding to the data bag, and if the data bag is labeled with the second state, the extraction module may input data instances to the auto-encoder in the order from a data instance which has the highest probabilities where it is in the second state to a data instance which has the lowest probabilities where it is in the second state and determine top part of data instances determined by the auto-encoder as being in the second state, as an instance for training corresponding to the data bag.

According to one of the other aspects of the present disclosure, provided is a neural network training system includes a storage module which stores an auto-encoder configured to determine whether an inputted patch is in a first state or a second state-here, the patch is one of those into which an image is segmented in a certain size, wherein the auto-encoder is pre-trained only with a plurality of patches which are in the first state; and a neural network which may output probabilities where the inputted patch is in the first state or the second state; an extraction module of extracting a patch for training which is part of patches forming an image for training, for each of a plurality of images for training labeled with any one of the first state or the second state; and a training module of training the neural network based on the patch for training corresponding to each of the plurality of images for training, wherein the extraction module is configured to input each patch forming the image for training to the neural network in training, calculate probabilities for each patch forming the image for training, and determine part of each patch forming the image for training as a patch for training based on probabilities for each patch forming the image for training and a determination result of the auto-encoder with respect to at least part of each patch forming the image for training.

In an example, the auto-encoder is pre-trained only with a patch which is in the first state, wherein in order to determine part of each patch forming the image for training as a patch for training based on probabilities for each patch forming the image for training and the determination result of the auto-encoder with respect to at least part of each patch forming the image for training, if the image for training is labeled with the first state, the extraction module is configured to input patches to the auto-encoder in the order from a patch which has the highest probabilities where it is in the second state to a patch which has the lowest probabilities where it is in the second state and determine top part of patches determined by the auto-encoder as being in the first state, as a patch for training corresponding to the image for training, and if the image for training is labeled with the second state, the extraction module is configured to input patches to the auto-encoder in the order from a patch which has the highest probabilities where it is in the second state to a patch which has the lowest probabilities where it is in the second state and determine top part of patches determined by the auto-encoder as being in the second state, as a patch for training corresponding to the image for training.

### ADVANTAGEOUS EFFECTS

According to a technical spirit of the present disclosure, many data instances, instances for training, may be extracted from one data bag. In the conventional multiple instance training method, if many instances for training are extracted from one data bag, there may be high probabilities to extract a wrong training data instance, and thus there may be a negative effect on training of the neural network. However, using a method according to the technical spirit of the present disclosure, there is an effect in significantly reducing extraction of the wrong training data instance by using the pre-trained auto-encoder and filtering an instance for training. Therefore, there is an effect of effectively training the neural network only with less data.

### BRIEF DESCRIPTION OF THE DRAWINGS

In order to more fully understand drawings cited in the detailed description of the present disclosure, brief description of each drawing is provided.
FIG. 1A is a view illustrating an example of training data used in the multiple instance training.
FIG. 1B is a view illustrating pseudo code which presents an example of a process of training a neural network via a multiple instance training technique.
FIG. 2 is a view illustrating schematic configurations of a computing system for performing a neural network training method according to the technical spirit of the present disclosure.
FIG. 3 is a view schematically illustrating a structure of an auto-encoder used in the neural network training method according to the technical spirit of the present disclosure.
FIG. 4 is a view illustrating an example of a method by which the neural network training system extracts data instances for training according to the technical spirit of the present disclosure.
FIG. 5 is a view illustrating an example of a specific process of the step S120 of FIG. 4.
FIG. 6 is a view illustrating an example of a specific process of the step S121 of FIG. 5.
FIG. 7 is a view illustrating schematic configurations of a determination system using the neural network according to an example of the present disclosure.
FIG. 8 illustrates an example in which the determination system using the neural network according to an example of the present disclosure outputs a lesion area within a pathological slide image in a form of a lattice map.

### BEST MODE FOR CARRYING OUT THE INVENTION

Various transformations applies to the present disclosure and there may be various examples, so that specific examples are exemplified in drawings and are specifically described in the detailed description. However, the above is not intended to limit a specific exemplary form to the present disclosure and should be understood as including all of transformations, equivalents, and substitutes included in the spirit and the technical range of the present disclosure. When describing the present disclosure, if it is determined that the detailed description of the disclosed relevant technologies may make the gist of the present disclosure vague, the detailed description thereof is omitted.

Terms such as first and second may be used for explaining various components, but the components should not be limited by the terms. The terms is only used for a purpose of distinguishing one component form another component.

Terms used in the present application is only used for explaining a specific example and is not intended to limit the present disclosure. A singular expression includes a plural expression as long as there is no definitely different meaning in the context.

In specification, it should be understood that the term such as "include" or "have" is to indicate that features, numbers, steps, operations, components, parts, or combinations thereof described in the specification are present and does not priorly exclude possibilities where one or more other features, numbers, steps, operations, components, parts, or combinations thereof may be present or be added.

Also, in the specification, if any one component 'transfers' data to another component, it means that the component may directly transfer the data to another component and may also transfer the data to another component via at least one other component. On the contrary, if any one component 'directly transfers' data to another component, it means that the data is transferred from one component to another component without the other component.

Hereinafter, referring to the attached drawings, the present disclosure is specifically described focusing on examples of the present disclosure. The same reference numeral presented in each drawing indicates the same component.

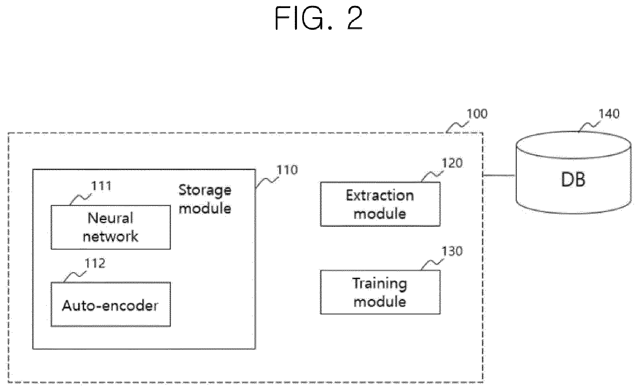

FIG. 2 is a view illustrating schematic configurations of a computing system for performing a neural network training method according to the technical spirit of the present disclosure.

The neural network training method according to the technical spirit of the present disclosure may be performed by a neural network training system 100.

The neural network training system 100 may be a computing system which is a data processing device having an arithmetic operation ability for realizing the technical spirit of the present disclosure and may include not only a server which is a data processing device to which a client may be connected via a network in general but also a computing device such as a personal computer or a portable terminal.

Those skilled in the art may easily infer that the neural network training system 100 may be implemented as any one physical device but a plurality of physical devices may be organically combined as required to implement the neural network training system 100 according to the technical spirit of the present disclosure.

Referring to FIG. 2, the neural network training system 100 may include a storage module 110, an extraction module 120, and a training module 130. According to an example of the present disclosure, some components of the above components may not correspond to components which are necessarily required for implementing the present disclosure. Also, according to an example, it is natural that the neural network training system 100 may include more components than the above. For example, the system 100 may further include a control module(not shown) for controlling a function and/or a resource of other components(e.g., the storage module 110, the extraction module 120, the training module 130, and the like) of the neural network training system 100. Also, according to an example, the neural network training system 100 may further include a database(DB) (140) for storing various information and/or data required for realizing the technical spirit of the present disclosure.

The neural network training system 100 may mean a logical configuration having a hardware resource and/or software required for realizing the technical spirit of the present disclosure and does not necessarily mean one physical component or one device. That is, the system 100 may mean a logical combination of hardware and/or software included for realizing the technical spirit of the present disclosure, and if required, it may be installed in spaced devices one another to perform each function, so that it may be implemented as a set of logical configurations for realizing the technical spirit of the present disclosure. Also, the system 100 may mean a set of configurations, which are separately implemented according to each function or role for realizing the technical spirit of the present disclosure. For example, each of the storage module 110, the extraction module 120, and the training module 130 may be located at different physical devices from one another and may be also located at the same physical device. Also, according to an embodiment, the combination of software and/or hardware forming each of the storage module 110, the extraction module 120, and the training module 130 may be also located at different physical devices from one another, wherein configurations located at different physical devices from one another may be organically combined to implement each of the modules.

Also, a module of the specification may mean a functional and structural combination of hardware and software for driving the hardware in order to perform the technical spirit of the present disclosure. For example, those skilled in the art may easily infer that the module may mean a logical unit of given code and a hardware resource for performing the given code, wherein it does not mean code which is necessarily physically connected or one type of hardware.

The storage module 110 may store a neural network 111 and an auto-encoder 112.

The neural network of the specification may include a multilayer perceptron model and may mean a set of information expressing a series of design considerations, which define an artificial neural network.

In an example, the neural network 112 may be a convolutional neural network. The convolutional neural network may include an input layer, a plurality of hidden layers, and an output layer as well known. Each of the plurality of hidden layers may include a convolutional layer and a pooling layer(or subsampling layer).

The convolutional neural network may be defined by a function, a filter, a stride, a weight factor, and the like for defining each of these layers. Also, the output layer may be defined as a fully connected FeedForward layer.

Design considerations for each layer forming the convolutional neural network are well known. For example, the number of layers to be included in a plurality of layers, a convolutional function for defining the plurality of layers, a pooling function, and known functions with respect to each activation function may be used, and separately defined functions for realizing the technical spirit of the present disclosure may be also used.

An example of the convolutional function is a discrete convolution sum, and the like. As an example of the pooling function, max pooling, average pooling, and the like may be used. An example of the activation function may be a sigmoid function, a tangent hyperbolic(tanh) function, a rectified linear unit(ReLU) function, and the like.

If the design considerations of the above convolutional neural network are defined, the convolutional neural network where design considerations are defined may be stored in a storing device. If the convolutional neural network is learned, a weight factor corresponding to each layer may be specified.

That is, training of the convolutional neural network may mean a process in which the weight factor of each layer is determined. Further, if the convolutional neural network is learned, the learned convolutional neural network may receive input data in an input layer and may output output data via the predetermined output layer.

The neural network according to an example of the present disclosure may be defined by selecting any one design consideration or a plurality of design considerations of well known design considerations as the above, and independent design considerations may be also defined for the neural network.

The neural network 111 may be a classification neural network which may be used for classification of the inputted data. Preferably, the neural network 111 may be a neural network which outputs probabilities about whether the inputted data is in a first state or a second state, thereby being used for binary classification with respect to the inputted data. For example, the neural network 111 may be a neural network for receiving a biometric image and determining probabilities that there is a lesion which occurs due to a certain disease(e.g., cancer) in the relevant image.

That is, the neural network 111 may output probabilities that a value inputted to the neural network 111 is in the given first state or second state. The first state may be any one of positive or negative, and the second state may be the remaining one of positive or negative. For example, the first state may be a normal state(negative) where there is not a lesion, and the second state may be an abnormal state(positive) where there is a lesion.

Probabilities that the neural network 111 outputs may be a value calculated by a loss function(e.g., a mean squared error(MSE), a cross entropy error(CEE), or a function presenting a distance between two vectors(e.g., a Euclidean distance, a n-norm distance, a Manhattan distance, and the like), etc.) within the neural network 111.

The auto-encoder 112 is a neural network structure which is mainly used in an unsupervised training methodology. The auto-encoder 112 is an unsupervised machine training model having a form such that a dimension of inputted values is minimized and then is restored again, wherein it has a function of training features which values used in training have. More specifically, the auto-encoder 112 learns a function which makes an output value close to an input value, extracts a feature with respect to the input value via the encoder, and reconstructs the input value via a decoder.

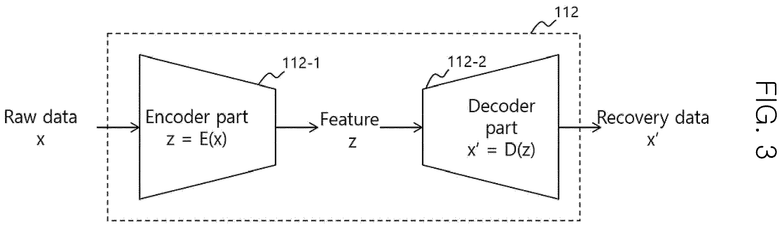

FIG. 3 is a view schematically illustrating a structure of the auto-encoder 112. Referring to FIG. 3, the auto-encoder 112 may include an encoder part 112-1 which includes a convolutional layer and a decoder part 112-2 which includes a deconvolutional layer. If raw data x is inputted to the encoder 111, encoding is performed with respect to the raw data x in the encoder part 112-1 and thus a feature(z=E(x)) of the raw data x may be generated. The generated z is decoded in the decoder part 112-2, so that recovery data(x'=D(z)) corresponding to the raw data x may be generated.

The auto-encoder is also a type of the neural network, so that training is preceded by many training data, wherein in a training step of the auto-encoder, the following steps 1) to 3) are performed, for each training data d.
1) The training data d is inputted to the auto-encoder 112 and goes through encoding and decoding processes, thereby generating recovery data d' corresponding to the training data d.
2) An error e=L(d, d') which is a difference between the training data d and the recovery data d' is calculated(where L is a loss function).
3) According to an error backpropagation method, a weighted value within the auto-encoder 112 is renewed.

Meanwhile, the auto-encoder 112 may be used for determining whether the inputted value is in the first state or the second state.

In an example, the auto-encoder 112 may be pre-trained only with the training data being in the first state, wherein when a given predicted target value is inputted to the auto-encoder 112, if there is a difference of a given limit value or more between a result value which the auto-encoder 112 restores(i.e., outputs) and the predicted target value, the predicted target value may be determined as being in the second state.

In one of other examples, the auto-encoder 112 may be pre-trained only with the data being in the second state, wherein when the given predicted target value is inputted to the learned auto-encoder 112, if there is a difference of a given limit value or more between a result value which the auto-encoder 112 restores(i.e., outputs) and the predicted target value, the predicted target value may be determined as being in the first state.

Meanwhile, the auto-encoder 112 according to the example may include a Variational AutoEncoder(VAE).

Again, referring to FIG. 2, the DB 140 may store training data to be used in training of the neural network 111. The training data may be data for multiple instance training as described with reference to FIG. 1A. That, each training dat stored in the DB(140) may a data bag including many data instances.

In an example, each of a plurality of training data may be a whole image, and a data instance forming each training data may be each image patch into which the whole image is segmented in a certain size. For example, each of the training data may be an intact pathological slide image. In this case, the data bag becomes one intact pathological slide image, the data instance included in the data bag may be an individual patch into which the pathological slide image is segmented in a certain size.

Meanwhile, the training data stored in the DB 140 may be labeled with the first state or the second state, respectively. For example, if the plurality of training data is the pathological slide image, each training data is labeled with a diagnosis result(e.g., whether there is a lesion, etc.) with respect to the pathological slide image.

Meanwhile, according to an example, the training data may not be stored in the DB 140 but may be inputted from a user via an external input means and may be also stored in a form of a file in a storing device such as HDD or SDD.

The extraction module 120 may perform an extracting step of extracting part of data instances included in the data bag as an instance for training, for each of a plurality of data bags labeled with any one of the first state or the second state. The instance for training extracted by the extraction module 120 may be used for training of the neural network 111 later.

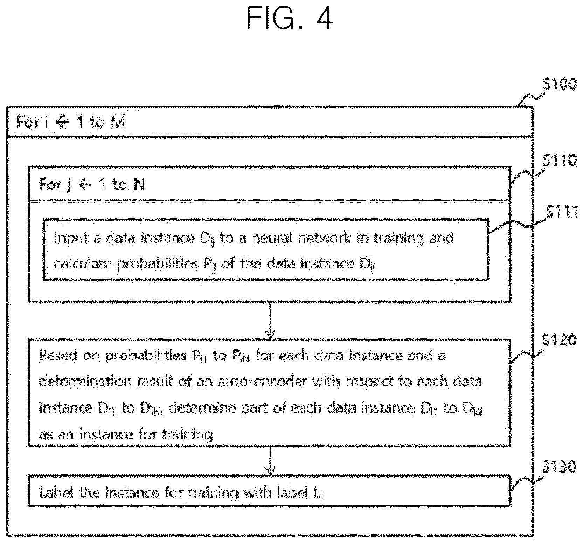

FIG. 4 is a view illustrating an example of a method that the extraction module 120 extracts a data instance for training. FIG. 4 exemplifies the case that data for training is as shown in FIG. 1A.

Referring to FIG. 4, the extraction module 120 may perform steps S110 to S130, for each of data bags B₁ to B_{M} in advance (S100).

Meanwhile, the extraction module 120 may input each data instance Dᵢⱼ included in the data bag Bᵢ to the neural network 111, with respect to the data bag Bᵢ(i is any integer where 1<=i<=M) (j is any integer where 1<=j<=N), and may calculate probabilities Pᵢⱼ of each data instance Dᵢⱼ included in the data bag Bᵢ(S110, S111). For example, Pᵢⱼ may be probabilities to be in the second state, and a Cross-entropy Loss with respect to data instance Dᵢⱼ may be calculated as probabilities Pᵢⱼ.

Thereafter, based on probabilities Pᵢ₁ to P_{iN} for each data instance included in the data bag Bᵢ, and a determination result of the auto-encoder 112 with respect to at least part of each data instance Dᵢ₁ to D_{iN} included in the data bag, the extraction module 120 may determine part of each data instance Dᵢ₁ to D_{iN} included in the data bag Bᵢ as an instance for training(S120) and may label the determined instance for training with label Lᵢ of Bᵢ(S130).

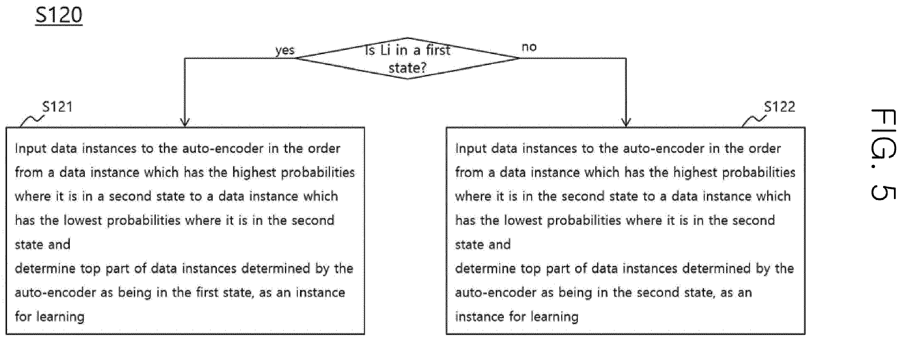

FIG. 5 is a view illustrating an example of a specific process of the step S120 of FIG. 4. In an example of FIG. 5, it is assumed that the auto-encoder 112 is pre-trained only with the data instance which is in the first state.

Referring to FIG. 5, if the data bag is labeled with the first state, the extraction module 120 may input data instances to the auto-encoder 112 in the order from a data instance which has the highest probabilities where it is in the second state to a data instance which has the lowest probabilities where it is in the second state and may determine top part of data instances determined by the auto-encoder as being in the first state, as an instance for training corresponding to the data bag(S121). Here, if a difference between the data instance inputted to the auto-encoder 112 and output data outputted by the auto-encoder 112 is a given limit value or more, the extraction module 120 may determine the data instance inputted to the auto-encoder 112 as being in the first state.

Meanwhile, if the data bag is labeled with the second state, the extraction module 120 may input data instances to the auto-encoder 112 in the order from a data instance which has the highest probabilities where it is in the second state to a data instance which has the lowest probabilities where it is in the second state and may determine top part of data instances determined by the auto-encoder 112 as being in the second state, as an instance for training corresponding to the data bag(S122).

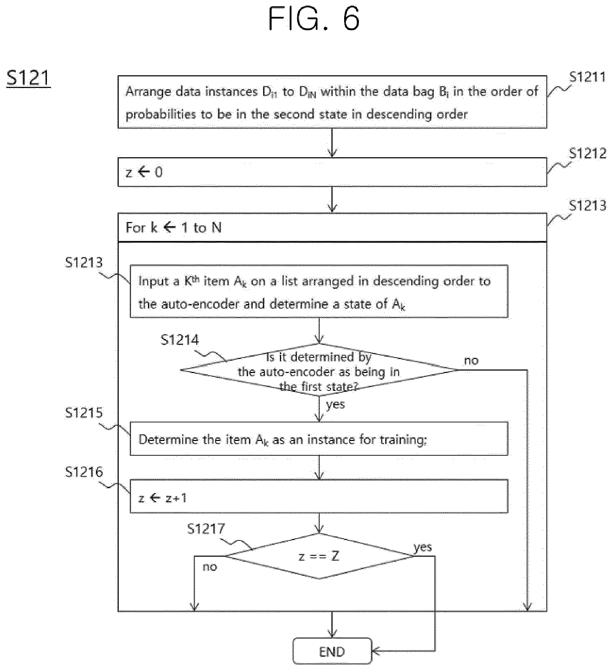

FIG. 6 is a view illustrating an example of a specific process of the step S121 of FIG. 5.

Referring to FIG. 6, the extraction module 120 may arrange data instances Dᵢ₁ to D_{iN} within the data bag Bᵢ in the order of probabilities to be in the second state in descending order(S1211).

The extraction module 120 may input a data instance Aₖ within Bᵢ to the auto-encoder 112 in the arranged order via the step S1211(k is any integer where 1<=j<=N) and may determine a state of the inputted data instance Aₖ(S1213). If the data instance Aₖ is determined as being in the first state, the data instance Aₖ may be determined as an instance for training(S1215).

The extraction module 120 may perform the steps S1213 to S1215 until a loop is completed or an instance for training corresponding to the data bag Bᵢ is found as many as the predetermined number 2(refer to S1212, S1216, and S1217).

Those skilled in the art may easily conceive a specific example of the step S122 of FIG. 5 referring to FIG. 6, so that the detailed description thereof is omitted. Meanwhile, it is natural that FIG. 5 and FIG. 6 are examples implementing the step S120 of FIG. 4, wherein there may be various methods implementing the step S120 of FIG. 4.

Via an extracting process as the above, the extraction module 120 may extract many data instances from one data bag as instances for training. In the conventional multiple instance training method, if many instances for training are extracted from one data bag, there are high probabilities to extract a wrong training data instance, and thus there is a negative effect on training of the neural network. However, according to the technical spirit of the present disclosure, there is an effect in significantly reducing extraction of a wrong training data instance by using the auto-encoder pre-trained with data instances for training only having any one state.

Again, referring to FIG. 2, the training module 130 may learn the neural network 111 based on an instance for training extracted by the extraction module 120.

The training module 130 may learn the neural network 111 by back-propagating a loss error between an instance for training inputted to the neural network 111 and an output value to the neural network 111.

The neural network training method considers a process of extracting a training data instance performed by the extraction module and a training process performed by the training module 130 as one epoch and repetitively performs them as many as a plurality of epochs, thereby enhancing performance of the neural network 111.

Meanwhile, the neural network training method according to the technical spirit of the present disclosure may apply to learn the neural network for disease diagnosis based on an image which may be used for diagnosis support for providing help to disease diagnosis or doctor's diagnosis based on an image. Hereinafter, an application example is described.

In the application example, the neural network 111 may be a neural network for diagnosis or diagnosis support which receives image patches into which a whole-slide-image is segmented in a certain size and determines whether there is a lesion due to a certain disease in the image patches. In this case, the DB 140 may store a plurality of pathological image slides. A pathological slide image may be various biometric images including a tissue image. Meanwhile, each pathological slide image may be labeled with any one of a first state (normal state) where there is not lesion or a second state(abnormal state) where there is a lesion. Meanwhile, in this application example, the auto-encoder 112 may be pre-trained only with an image patch being in the normal state where there is no lesion. For example, a learner may select only one labeled with the normal state of pathological slide images stored in the DB 140 to learn the neural network 111, may segment the same into patches, and then may pre-learn the auto-encoder 112. Also, the learner may collect a patch which is not used for training the neural network 111 and is separately in the normal state, and may pre-learn the auto-encoder 112.

In this application example, the extraction module 120 may perform an extracting step of extracting a patch for training which is part of patches forming a pathological slide image for training, for each of a plurality of pathological slide images for training labeled with any one of the normal state or the abnormal state. The training module 130 may perform a training step of training the neural network 111 based on the extracted patch for training.

Here, in the extracting step, the extraction module 120 may input each patch forming the pathological slide image for training to the neural network 111 in training, may calculate probabilities for each patch forming the pathological slide image for training, and may determine part of each patch forming the pathological slide image for training as a patch for training based on probabilities for each patch forming the pathological slide image for training and a determination result of the auto-encoder 112 with respect to at least part of each patch forming the pathological slide image for training.

More specifically, if the pathological slide images for training are labeled with the normal state, the extraction module 120 may input patches to the auto-encoder 112 in the order from a patch which has the highest probabilities where it is in the abnormal state to a patch which has the lowest probabilities where it is in the abnormal state and may determine top part of patches determined by the auto-encoder 112 as being in the normal state, as a patch for training corresponding to the image for training. Also, if the image for training is labeled with the abnormal state, the extraction module 120 may input patches to the auto-encoder 112 in the order from a patch which has the highest probabilities where it is in the abnormal state to a patch which has the lowest probabilities where it is in the abnormal state and may determine top part of patches determined by the auto-encoder 112 as being in the abnormal state, as a patch for training corresponding to the image for training.

Meanwhile, the neural network learned by the neural network training method may be loaded in a certain determination system and may perform prediction about the inputted data. In an example, the neural network loaded in the determination system may be used for determination to an image. That is, the neural network may be for determining whether the given image is in the first state or the second state. Meanwhile, the determination system may be a certain computing system.

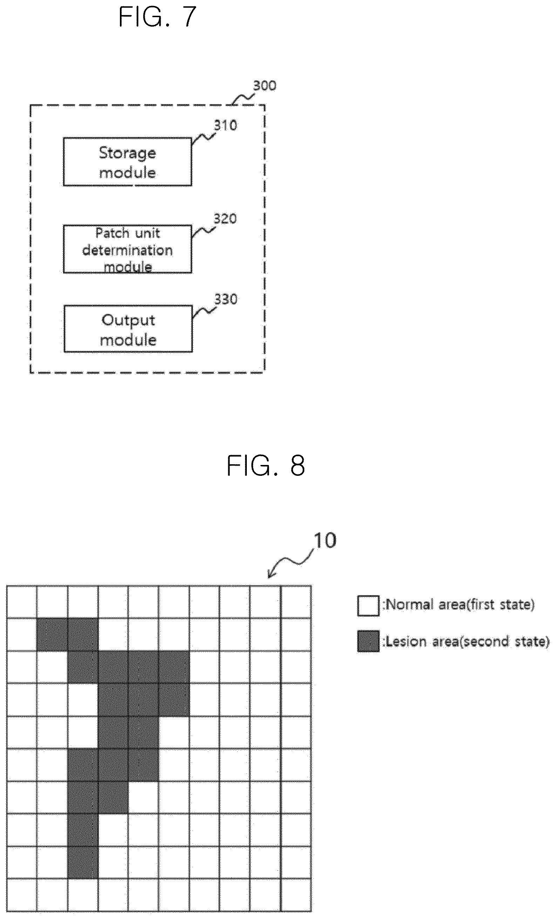

Hereinafter, an example of the determination system equipped with the neural network learned by the above neural network training method is described with reference to FIG. 7.

FIG. 7 is a view illustrating schematic configurations of a determination system using the neural network according to an example of the present disclosure.

Referring to FIG. 7, a determination system 300 using the neural network may include a storage module 310, a patch unit termination module 320, and an output module 330.

The storage module 310 may store the neural network learned by the neural network training method. The storage module 310 may be a storing means which may store various information and data including the neural network.

The patch unit determination module 320 may input each of a plurality of diagnosis patches into which the given determination target image is segmented to the neural network and may obtain a determination result corresponding to each of the plurality of diagnosis patches.

Based on the determination result of each of the plurality of diagnosis patches obtained by the patch unit determination module, the output module 330 may output a lattice map of the determination target image. The lattice map may mean a map which may visually distinguish a patch area being in the first state from a patch area being in the second state.

The determination system() using the neural network may be used as a system which receives a pathological slide image and outputs a lesion area. In this case, the determination system 300 may receive a pathological slide image including a lesion due to a certain disease. The patch unit determination module 320 may segment the pathological slide image into patches and may determine whether there is a lesion for each patch. Thereafter, based on the determination result for each patch, the output module() may output the lesion area of the pathological slide image in a form of a lattice map distinguished by a patch.

FIG. 8 illustrates an example in which a lesion area within the pathological slide image is outputted in a form of a lattice map. In FIG. 8, an area (lesion area) corresponding to a patch determined as being in the second state is presented with dark color.

Meanwhile, the computing device 100 may include a processor and a storing device. The processor may mean an arithmetic device which may drive a program for realizing the technical spirit of the present disclosure and may perform a neural network training method defined by the program and the technical spirit of the present disclosure. The processor may include a single core CPU or a multi-core CPU. The storing device may mean a data storing means which may store a program and various data required for realizing the technical spirit of the present disclosure and may be implemented as a plurality of storing means according to an embodiment. Also, the storing device may have the meaning which includes not only a main memory included in the computing device 100 but also transient storage or a memory, and the like which may be included in the processor. A memory may include a high-speed random access memory and may also include a non-volatile memory such as one or more magnetic disk storage device, flash memory device, or other non-volatile solid state memories. Access to the memory by the processor and other components may be controlled by a memory controller.

Meanwhile, a method according to an example of the present disclosure may be implemented in a form of a program instruction which may be read by a computer and may be stored in a computer-readable recording medium. A control program and a target program according to an example of the present disclosure may be also stored in the computer-readable recording medium. The computer-readable recording medium may include all types of recording devices where data which may be read by a computer system is stored.

The program instruction recorded on the recording medium is one which is specially designed and configured for the present disclosure or one which is known to those skilled in the art of a software field and is usable.

Examples of the computer-readable recording medium include magnetic media such as a hard disk, a floppy disk, and a magnetic tape, optical media such as CD-ROM and DVD, magneto-optical media such as a floptical disk, a hardware device which is specially configured to store and perform program instructions such as ROM, RAM, and a flash memory. Also, the computer-readable recording medium may store and run code which may be distributed on a computer system connected to a network and may be read by a computer in a distributed way.

Examples of the program instruction include not only machine language code made by a compiler but also high-level language code which may be run by a device, which electronically processes information using an interpreter and the like, e.g., a computer.

The above hardware device may be configured to operate as one or more software module for performing an operation of the present disclosure, and vice versa.

The above description of the present disclosure is for examples, and those skilled in the art may understand that the above may be easily changed to other specific variations without changing the technical spirit of the present disclosure or necessary features. Therefore, it is to be understood that the examples described above are exemplary in all aspects and are not limited. For example, each component described as a single type may be implemented to be distributed. In the same way, components described as being distributed may be implemented in a combined form.

The scope of the present disclosure is presented by the scope of patent claims to be described hereinafter more than the above detailed description. All changes or variations conceived from meanings of the scope of patent claims and ranges and concepts of equivalents thereof is to be interpreted as being included in the scope of the present disclosure.

### Industrial Applicability

The present disclosure may be used for a neural network training method via an auto-encoder and multiple instance training, and a computing system for performing the same.

## Claims

1. A neural network training method performed in a computing system which includes an auto-encoder for determining whether an inputted data instance is in a first state or a second state and a neural network which outputs probabilities where the inputted data instance is in the first state or the second state, wherein the neural network training method comprises:
an extracting step of, for each of a plurality of data bags labeled with any one of the first state or the second state, extracting an instance for training which is part of data instances included in the data bag, ; and
a training step of training the neural network based on an instance for training corresponding to each of the plurality of data bags, wherein the extracting step includes:
a step of inputting each data instance included in the data bag to the neural network in training and calculating probabilities for each data instance included in the data bag; and
a step of determining part of each data instance included in the data bag as an instance for training based on probabilities for each data instance included in the data bag and a determination result of the auto-encoder with respect to at least part of each data instance included in the data bag.

2. The neural network training method of claim 1, wherein the computing system is configured to determine whether the data instance inputted to the auto-encoder is in the first state or the second state, based on a difference between the data instance inputted to the auto-encoder and output data outputted from the auto-encoder.

3. The neural network training method of claim 1, wherein the auto-encoder is pre-trained only with a data instance which is in the first state, wherein the step of determining part of each data instance included in the data bag as an instance for training based on probabilities for each data instance included in the data bag and the determination result of the auto-encoder with respect to at least part of each data instance included in the data bag includes:
a step of if the data bag is labeled with the first state, inputting data instances to the auto-encoder in the order from a data instance which has highest probabilities where it is in the second state to a data instance which has lowest probabilities where it is in the second state and determining top part of data instances determined by the auto-encoder as being in the first state, as an instance for training corresponding to the data bag; and
a step of if the data bag is labeled with the second state, inputting data instances to the auto-encoder in the order from a data instance which has the highest probabilities where it is in the second state to a data instance which has the lowest probabilities where it is in the second state and determining top part of data instances determined by the auto-encoder as being in the second state, as an instance for training corresponding to the data bag.

4. The neural network training method of claim 1, further comprising a step of repetitively performing 1 epoch or more of the extracting step and the training step.

5. The neural network training method of claim 1, wherein each of the plurality of data bags is a whole image, and a data instance included in each of the plurality of data bags is each image patch into which the whole image corresponding to the data bag is segmented in a certain size.

6. A neural network training method performed in a computing system which includes an auto-encoder for determining whether an inputted patch is in a first state or a second state-here, the patch is one of those into which an image is segmented in a certain size-; and a neural network which outputs probabilities where the inputted patch is in the first state or the second state, wherein the neural network training method comprises:
an extracting step of, for each of a plurality of images for training labeled with any one of the first state or the second state, extracting a patch for training which is part of patches forming the image for training; and
a training step of training the neural network based on the patch for training corresponding to each of the plurality of images for training, wherein the extracting step includes:
a step of inputting each patch forming the image for training to the neural network in training, and calculating probabilities for each patch forming the image for training; and
a step of determining part of each patch forming the image for training as a patch for training based on probabilities for each patch forming the image for training and a determination result of the auto-encoder with respect to at least part of each patch forming the image for training.

7. The neural network training method of claim 6, wherein the auto-encoder is pre-trained only with a patch which is in the first state, wherein the step of determining part of each patch forming the image for training as a patch for training based on probabilities for each patch forming the image for training and the determination result of the auto-encoder with respect to at least part of each patch forming the image for training includes:
a step of if the image for training is labeled with the first state, inputting patches to the auto-encoder in the order from a patch which has highest probabilities where it is in the second state to a patch which has lowest probabilities where it is in the second state and determining top part of patches determined by the auto-encoder as being in the first state, as a patch for training corresponding to the image for training; and
a step of if the image for training is labeled with the second state, inputting patches to the auto-encoder in the order from a patch which has the highest probabilities where it is in the second state to a patch which has the lowest probabilities where it is in the second state and determining top part of patches determined by the auto-encoder as being in the second state, as a patch for training corresponding to the image for training.

8. The neural network training method of claim 6, wherein each of images for training is any one of an image including a lesion due to a certain disease or an image not including the lesion, the first state is a normal state where the lesion is not present, and the second state is an abnormal state where the lesion is present.

9. A determination system using a neural network, comprising:
a storage module configured to store the neural network trained by the neural network training method described in claim 6;
a patch unit determination module configured to input each of a plurality of diagnosis patches into which a given determination target image is segmented to the neural network and obtain a determination result corresponding to each of the plurality of diagnosis patches; and
an output module configured to output a heat map of a determination target image based on a determination result of each of the plurality of diagnosis patches obtained by the patch unit diagnosis module.

10. A computer program installed in a data processing device and recorded on a medium for performing the method described in any of claims 1 to 8.

11. A computer readable recording medium on which a computer program for performing the method described in any of claims 1 to 8 is recorded.

12. A computing system, comprising: a processor and a memory, wherein if the memory is performed by the processor, the computing system performs the method described in any of claims 1 to 8.

13. A neural network training system, comprising:
a storage module configured to store an auto-encoder for determining whether an inputted data instance is in a first state or a second state and a neural network which outputs probabilities where the inputted data instance is in the first state or the second state;
an extraction module configured to, for each of a plurality of data bags labeled with any one of the first state or the second state, extract an instance for training which is part of data instances included in the data bag, ; and
a training module of training the neural network based on the instance for training corresponding to each of the plurality of data bags, wherein
the extraction module is configured to input each data instance included in the data bag to the neural network in training, calculate probabilities for each data instance included in the data bag, and determine part of each data instance included in the data bag as an instance for training based on probabilities for each data instance included in the data bag and a determination result of the auto-encoder with respect to at least part of each data instance included in the data bag.

14. The neural network training system of claim 13, wherein the extraction module is configured to determine whether the data instance inputted to the auto-encoder is in the first state or the second state, based on a difference between the data instance inputted to the auto-encoder and output data outputted from the auto-encoder.

15. The neural network training system of claim 12, wherein an auto-encoder is pre-trained only with a data instance which is in a first state, wherein
in order to determine part of each data instance included in a data bag as an instance for training based on probabilities for each data instance included in the data bag and a determination result of the auto-encoder with respect to at least part of each data instance included in the data bag,
if the data bag is labeled with the first state, the extraction module is configured to input data instances to the auto-encoder in the order from a data instance which has highest probabilities where it is in a second state to a data instance which has lowest probabilities where it is in the second state and determine top part of data instances determined by the auto-encoder as being in the first state, as an instance for training corresponding to the data bag, and
if the data bag is labeled with the second state, the extraction module is configured to input data instances to the auto-encoder in the order from a data instance which has the highest probabilities where it is in the second state to a data instance which has the lowest probabilities where it is in the second state and determine top part of data instances determined by the auto-encoder as being in the second state, as an instance for training corresponding to the data bag.

16. A neural network training system, comprising:
a storage module which stores:
an auto-encoder for determining whether an inputted patch is in a first state or a second state-here, the patch is one of those into which an image is segmented in a certain size, wherein the auto-encoder is pre-trained only with a plurality of patches which are in the first state; and
a neural network which outputs probabilities where the inputted patch is in the first state or the second state;
an extraction module configured to, for each of a plurality of images for training labeled with any one of the first state or the second state, extract a patch for training which is part of patches forming the image for training ; and
a training module configured to train the neural network based on the patch for training corresponding to each of the plurality of images for training, wherein
the extraction module is configured to input each patch forming the image for training to the neural network in training, calculate probabilities for each patch forming the image for training, and determine part of each patch forming the image for training as a patch for training based on probabilities for each patch forming the image for training and a determination result of the auto-encoder with respect to at least part of each patch forming the image for training.

17. The neural network training system of claim 16, wherein the auto-encoder is pre-trained only with a patch which is in the first state, wherein
in order to determine part of each patch forming the image for training as a patch for training based on probabilities for each patch forming the image for training and the determination result of the auto-encoder with respect to at least part of each patch forming the image for training, an extraction module is configured to:
if the image for training is labeled with the first state, input patches to the auto-encoder in the order from a patch which has highest probabilities where it is in the second state to a patch which has lowest probabilities where it is in the second state and determine top part of patches determined by the auto-encoder as being in the first state, as a patch for training corresponding to the image for training; and
if the image for training is labeled with the second state, input patches to the auto-encoder in the order from a patch which has the highest probabilities where it is in the second state to a patch which has the lowest probabilities where it is in the second state and determine top part of patches determined by the auto-encoder as being in the second state, as a patch for training corresponding to the image for training.
